(19) Europäisches Patentamt / European Patent Office / Office européen des brevets

(11) **EP 3 546 936 A1**

(12) **EUROPEAN PATENT APPLICATION**
published in accordance with Art. 153(4) EPC

(43) Date of publication:
**02.10.2019 Bulletin 2019/40**

(51) Int Cl.:
*G01N 33/497* [(2006.01)]    *G01N 33/66* [(2006.01)]

(21) Application number: **17873872.0**

(22) Date of filing: **24.11.2017**

(86) International application number:
**PCT/JP2017/042150**

(87) International publication number:
**WO 2018/097222 (31.05.2018 Gazette 2018/22)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **25.11.2016 JP 2016229121**

(71) Applicant: **Otsuka Pharmaceutical Co., Ltd. Tokyo 101-8535 (JP)**

(72) Inventors:
• **INADA, Makoto**
 **Osaka-shi**
 **Osaka 540-0021 (JP)**

• **KAWATA, Keiko**
 **Osaka-shi**
 **Osaka 540-0021 (JP)**
• **SUDOU, Kimiyoshi**
 **Osaka-shi**
 **Osaka 540-0021 (JP)**

(74) Representative: **dompatent von Kreisler Selting Werner - Partnerschaft von Patent- und Rechtsanwälten mbB Deichmannhaus am Dom Bahnhofsvorplatz 1 50667 Köln (DE)**

(54) **NUTRITIONAL STATE EVALUATION METHOD**

(57)    An aspect of the disclosure provides a method of evaluating the nutriture of a subject, comprising (1) calculating the ratio of the $^{13}CO_2$ amount to the unlabeled $CO_2$ amount or total $CO_2$ amount in an expired air sample obtained from the subject to which a $^{13}C$-labeled amino acid was administered; and (2) comparing the ratio with a reference value to evaluate the nutriture. Another aspect of the disclosure provides a composition comprising a $^{13}C$-labeled amino acid for evaluating the nutriture of a subject.

EP 3 546 936 A1

**Description**

TECHNICAL FIELD

This application claims the benefit of priority of Japanese Patent Application No. 2016-229121, the entire contents of which are incorporated herein by reference.

The disclosure relates to a method of evaluating the nutriture of a subject and a composition for use in the method.

BACKGROUND ART

Evaluating nutriture is useful for maintaining appropriate nutriture or improving nutriture. Nutriture is generally evaluated using a static nutritional index (e.g., blood albumin level, body measurement, or triceps skin fold thickness) or a dynamic nutritional index (e.g., rapid turnover protein (RTP), resting energy expenditure (REE), or respiratory quotient (RQ)). Static nutritional indices reflect long-term nutriture and cannot evaluate short-term changes in nutriture. In contrast, dynamic nutritional indices reflect short-term and real-time nutriture.

The respiratory quotient, a dynamic nutritional index, is the volume ratio of carbon dioxide produced by the body to oxygen consumed by the body during catabolism of nutrients and conversion into energy. Because the respiratory quotients for carbohydrates, lipids, and proteins are known to be 1.0, about 0.7, and about 0.8, respectively, the type of the nutrient being catabolized in the body can be derived by measuring the respiratory quotient of the subject. Respiratory quotients are high under the conditions where the food intake is sufficient and glycogen is stored in the liver, because carbohydrate is mainly catabolized under such conditions. In contrast, respiratory quotients are low under the conditions where the food intake is insufficient or glycogen cannot be stored in the liver, because lipids and/or proteins are catabolized under such conditions. The respiratory quotient measurement thus can be used for evaluating the nutriture of a subject.

However, respiratory quotients can be measured only in the limited facilities, because an indirect calorimeter, a special and expensive apparatus, is required. The measurement with an indirect calorimeter usually takes 2 hours or more. During the measurement, the patient has to lie and rest, but is forbidden to sleep. This requires a supervisor who observes whether the respiratory quotient is measured under the appropriate conditions. Such situation gives pain and burdens to both the patient and the supervisor.

Accordingly, an alternative method to the respiratory quotient measurement for evaluating the dynamic nutriture has been demanded. For example, a method for evaluating the energy malnutrition in a test subject having a liver disease comprising administering isotope-labeled glucose to the subject and measuring the isotope contained in an expired air (Patent Literature 1) and a method for measuring a sugar/fatty acid combustion ratio in a test subject using isotope-labeled glucose and/or fatty acid (Patent Literature 2 and 3) have been proposed. However, no isotope-labeled amino acid has been used for evaluating the nutriture of a subject.

REFERENCES

PATENT LITERATURE

[Patent Literature 1] WO 2016/006601
[Patent Literature 2] WO 2014/030650
[Patent Literature 3] WO 2014/142248

SUMMARY OF THE INVENTION

A method for simply and rapidly evaluating the real-time nutriture of a subject has been demanded.

The inventors have found that the nutriture of a subject can be simply and rapidly evaluated by administering a $^{13}$C-labeled amino acid to the subject, collecting an expired air sample, and observing the behavior of labeled carbon dioxide ($CO_2$) in the sample, especially the abundance of carbon dioxide, i.e., the ratio of the labeled $CO_2$ amount to the unlabeled $CO_2$ amount or total $CO_2$ amount.

An aspect of the disclosure accordingly provides a method of evaluating the nutriture of a subject, comprising:

(1) calculating the ratio of the $^{13}CO_2$ amount to the unlabeled $CO_2$ amount or total $CO_2$ amount in an expired air sample obtained from the subject to which a $^{13}$C-labeled amino acid was administered; and
(2) comparing the ratio with a reference value to evaluate the nutriture.

An aspect of the disclosure provides a composition comprising a $^{13}C$-labeled amino acid for evaluating the nutriture of a subject.

The disclosure enables to evaluate the real-time nutriture of a subject more simply and rapidly than the conventional methods involving respiratory quotient measurement. Such evaluation can provide useful information for diagnosing a disease, e.g., a hepatic disease or a metabolic disease, and selecting therapy for such disease.

BRIEF DESCRIPTION OF DRAWINGS

Fig. 1 shows the respiratory quotients of ZDF lean rats in the periods of feeding and fasting.
Fig. 2 shows the $\Delta^{13}C$ (‰) values of the expired air samples obtained from fed and fasted ZDF lean rats to which 1-$^{13}C$-leucine, 1-$^{13}C$-alanine, or 1-$^{13}C$-glutamic acid was administered.
Fig. 3 shows the respiratory quotients of ZDF fatty rats F1 and F2.
Fig. 4 shows the $\Delta^{13}C$ (‰) values of the expired air samples obtained from ZDF lean rats F1 and F2 to which 1-$^{13}C$-alanine was administered, as well as their blood albumin levels.
Fig. 5 shows the respiratory quotients of ZDF fatty rats F1, F2, and F3.
Fig. 6 shows the $\Delta^{13}C$ (‰) values of the expired air samples obtained from ZDF lean rats F1 and F2 to which 1-$^{13}C$-glutamic acid was administered, as well as their blood albumin levels.

DETAILED DESCRIPTION

Unless otherwise defined, the terms used herein are read as generally understood by a skilled person in the technical fields such as organic chemistry, medicine, pharmacology, molecular biology, and microbiology. Several terms used herein are defined as described below. The definitions herein take precedence over the general understanding.

When a numerical value is accompanied with the term "about", the value is intended to represent any value within the range of $\pm$ 10% of that value. A numerical range covers all values from the lower limit to the upper limit and includes the values of the both limits. When a numerical range is accompanied with the term "about", the both limits are read as accompanied with the term. For example, "about 20 to 30" is read as "20 $\pm$ 10% to 30 $\pm$ 10%."

The "subject" may be any mammal the nutriture of which is to be evaluated. Examples thereof include humans and mammals other than humans. Examples of the mammals other than humans include mice, rats, guinea pigs, rabbits, dogs, cats, monkeys, swine, bovines, and horses, and preferable examples thereof include mice, rats, guinea pigs, rabbits, dogs, and monkeys.

The subject may be healthy, or may have a disease, e.g., a cancer, a hepatic disease, a metabolic disease, sarcopenia, or a lung disease. Examples of hepatic diseases include liver cirrhosis, non-alcoholic fatty liver disease (NAFLD), non-alcoholic steatohepatitis (NASH), viral hepatitis (e.g., hepatitis B or hepatitis C), alcoholic liver disease, primary biliary cirrhosis, primary sclerosing cholangitis, hemochromatosis, and autoimmune hepatitis, especially liver cirrhosis. Metabolic diseases include diseases associated with abnormal glycogen storage in the liver and diseases associated with abnormal energy metabolism, e.g., diabetes, borderline diabetes, and insulin resistance. Sarcopenia includes, e.g., primary sarcopenia and secondary sarcopenia. Lung diseases include, e.g., chronic obstructive pulmonary disease (COPD). The subject may be a subject suspected of having such a disease.

The subject may be in a fasted state or a fed state immediately before the administration of a $^{13}C$-labeled amino acid. For example, the subject may be in a fasted state, e.g., for at least 2 hours, preferably for at least 4 hours, immediately before the administration of a $^{13}C$-labeled amino acid, with freely ingesting water. In a fed state the subject may ingest usual diet as usual.

The subject may be under dietary restriction. The term "dietary restriction" means limiting the type and/or amount of nutrients to be ingested. Dietary restriction may be performed, for example, for treating or preventing a disease such as a liver disease or a metabolic disease, or for reducing weight for beauty. Dietary restriction provided for treating or preventing a disease is also referred to as dietary therapy. Dietary therapy also includes fasting therapy in which no nutrient is ingested for a period of time. Diet therapy is provided, for example, for treating and/or preventing a disease, e.g., diabetes, boundary diabetes, insulin resistance, high blood glucose, renal disease, hypertension, dyslipidemia, metabolic syndrome, obesity, hyperuricemia, gout, thromboembolism, ischemic heart disease, or arteriosclerosis.

The term "nutriture" herein means status related to nutrients as an energy source, including status of intake, retention, and metabolism of nutrients. Mammals mainly use carbohydrates as an energy source when food intake is sufficient and glycogen is stored in the liver. On the other hand, mammals use lipids and proteins as an energy source when food intake is insufficient, carbohydrates cannot be used because of insulin resistance, or no glycogen is stored in the liver. For example, the liver diseases and metabolic diseases mentioned above, especially liver cirrhosis, prevent glycogen storage in the liver. The term "normal nutriture" herein means the status wherein the subject mainly uses carbohydrates as the energy source. The term "undernutrition" herein means the status wherein the subject mainly uses lipids and/or proteins as the energy source.

The term "$^{13}$C-labeled amino acid" means an amino acid that can be metabolized by a mammal and is labeled with $^{13}$C in a manner such that at least a portion of the $CO_2$ formed through a metabolic pathway is labeled with $^{13}$C. Typically the carbon in the carboxyl group and/or the alpha carbon is $^{13}$C. A carbon in a side group that is converted to $CO_2$ through a metabolic pathway may be $^{13}$C. A carbon atom at one position in an amino acid molecule or carbon atoms at plural positions may be labeled. Preferably, a carbon atom at one position is labeled. More preferably, the carbon atom in the carboxyl group is labeled. Typically, a mixture comprising amino acids labeled with $^{13}$C at the same position or positions is administered to a subject. A mixture comprising amino acids labeled at different positions also may be administered.

Amino acids may be $^{13}$C-labeled by any of the commonly used methods without limitation (e.g., Yasuhito Sasaki, "5.1 Antei Doitai no Rinsho Shindan heno Oyo [5.1 Application of Stable Isotopes in Clinical Diagnosis]," Kagaku no Ryoiki [Journal of Japanese Chemistry] 107, pp. 149-163 (1975), Nankodo; Kajiwara, RADIOISOTOPES, 41, 45-48 (1992)). Isotope-labeled compounds, especially $^{13}$C-labeled amino acids used in the Examples below, are commercially available, and commercial products can be easily used.

Amino acids are categorized into glucogenic amino acids, ketogenic amino acids, or amino acids that are both glucogenic and ketogenic, in view of the metabolic pathways. The glucogenic amino acids are amino acids whose carbon skeletons after deamination are used for glycosylation. For example, the glucogenic amino acids in humans include amino acids which are converted via pyruvic acid to oxaloacetic acid and used for glycosylation (alanine, glycine, serine, threonine, cysteine, and tryptophan); amino acids which are converted to succinyl-CoA, then converted to oxaloacetic acid in the citric acid cycle, and used for glycosylation (isoleucine, methionine, valine, and threonine); amino acids which are converted to oxaloacetic acid and used for glycosylation (aspartic acid and asparagine); amino acids which are converted to alpha-ketoglutaric acid, then converted to oxaloacetic acid in the citric acid cycle, and used for glycosylation (arginine, glutamic acid, glutamine, histidine, and proline); and amino acids which are converted to fumaric acid, then converted to oxaloacetic acid in the citric acid cycle, and used for glycosylation (tyrosine, phenylalanine, and aspartic acid). The ketogenic amino acids are amino acids whose carbon skeletons after deamination can be converted to fatty acids or ketones via lipid metabolism pathways. For example, the human ketogenic amino acids include leucine, lysine, threonine, isoleucine, tyrosine, phenylalanine, and tryptophan. In humans, threonine, isoleucine, tyrosine, phenylalanine, and tryptophan are both glucogenic and ketogenic. One amino acid may be used, or two or more amino acids may be used in combination. In one embodiment, the $^{13}$C-labeled amino acid is a $^{13}$C-labeled glucogenic amino acid. In another embodiment, the $^{13}$C-labeled amino acid is a $^{13}$C-labeled ketogenic amino acid.

For example, the $^{13}$C-labeled amino acid may be selected from the group consisting of $^{13}$C-labeled alanine, glycine, serine, threonine, cysteine, tryptophan, isoleucine, methionine, valine, aspartic acid, asparagine, arginine, glutamic acid, glutamine, histidine, proline, tyrosine, phenylalanine, leucine, and lysine. For example, the $^{13}$C-labeled amino acid may be selected from the group consisting of $^{13}$C-labeled alanine, glycine, serine, threonine, cysteine, tryptophan, isoleucine, arginine, glutamic acid, glutamine, histidine, proline, tyrosine, phenylalanine, leucine, and lysine. For example, the $^{13}$C-labeled amino acid may be selected from the group consisting of $^{13}$C-labeled alanine, glutamic acid, and leucine. For example, the $^{13}$C-labeled amino acid may be selected from the group consisting of $^{13}$C-labeled alanine and glutamic acid. In one embodiment, the $^{13}$C-labeled amino acid is $^{13}$C-labeled alanine, glycine, serine, threonine, cysteine, or tryptophan, especially $^{13}$C-labeled alanine. In one embodiment, the $^{13}$C-labeled amino acid is $^{13}$C-labeled glutamic acid, glutamine, arginine, histidine, or proline, especially $^{13}$C-labeled glutamic acid. In one embodiment, the $^{13}$C-labeled amino acid is $^{13}$C-labeled leucine, lysine, threonine, isoleucine, tyrosine, phenylalanine, or tryptophan, especially $^{13}$C-labeled leucine.

The $^{13}$C-labeled amino acid may be administered to the subject as a free $^{13}$C-labeled amino acid, a part of a peptide or protein, or a pharmaceutically acceptable salt thereof. The $^{13}$C-labeled amino acid may be contained in a composition. Any composition may be used as long as $^{13}$C-labeled carbon dioxide is excreted into the expired air after the $^{13}$C-labeled amino acid is absorbed and metabolized in the body. The composition may consist of the $^{13}$C-labeled amino acid only or may comprise an additional component such as a carrier or an additive. The composition may have any feature, such as form, an additional component, proportion of each component, and a preparation method, without limitation. The composition is one of the aspects of the disclosure.

The composition may be in any form without limitation, for example, oral dosage form or parenteral dosage form. Examples of oral dosage forms include any oral dosage form, for example liquids such as solutions (including syrup), suspensions, and emulsions; and solids such as tablets (with or without coating), chewable tablets, capsules, pills, pulvis (powders), fine particles, and granules. Examples of parenteral dosage forms include dosage forms such as injections and drops (in liquid, suspension, or emulsion form). Preferred examples include an oral dosage form and an injectable dosage form, especially an intravenous dosage form. In an embodiment, the composition is in oral dosage form.

For example, a liquid composition, e.g., an injectable dosage form such as a liquid, suspension, or emulsion form, may be prepared by combining a $^{13}$C-labeled amino acid with a solvent such as physiological saline, distilled water for injection, or purified water. The composition may comprise a pharmaceutically acceptable carrier or additive which is routinely used in the field, for example, an isotonic agent (e.g., sodium chloride), a pH adjuster (e.g., hydrochloric acid or sodium hydroxide), a buffer (e.g., boric acid, sodium monohydrogen phosphate, or sodium dihydrogen phosphate), a preservative (e.g., benzalkonium chloride), and a thickener (e.g., carboxyvinyl polymers).

The composition may be in a solid form, such as a freeze-dried preparation or a spray-dried preparation that is dissolved in a solvent, e.g., a physiological saline, distilled water for injection, or purified water, before use.

Solid compositions, e.g., tablets, chewable tablets, capsules, pills, pulvis, fine particles, granules, may be prepared by combining a $^{13}$C-labeled amino acid with a component such as a carrier or an additive. The tablets may be coated with an ordinary coating to provide coated tablets, e.g., sugar-coated tablets, gelatin-coated tablets, film-coated tablets, double-coated tablets, and multi-coated tablets. The capsules may be prepared by a commonly employed method, i.e., mixing a labeled amino acid with a necessary component and filling capsules, such as hard gelatin capsules or soft capsules, with the mixture.

Examples of carriers or additives include lactose, sucrose, dextrin, mannitol, xylitol, sorbitol, erythritol, calcium dihydrogen phosphate, sodium chloride, glucose, urea, starch, calcium carbonate, kaolin, crystalline cellulose, silicic acid, water, ethanol, simple syrup, glucose liquid, starch liquid, gelatin liquid, carboxymethyl cellulose, sodium carboxymethyl cellulose, shellac, methyl cellulose, hydroxypropylmethyl cellulose, hydroxypropyl cellulose, potassium phosphate, polyvinyl alcohol, polyvinyl pyrrolidone, pullulan, dry starch, sodium alginate, agar powder, laminaran powder, sodium bicarbonate, polyoxyethylene sorbitan fatty acid esters, sodium lauryl sulfate, monoglyceride stearate, carmellose calcium, low substituted hydroxypropyl cellulose, carmellose, croscarmellose sodium, sodium carboxymethyl starch, crospovidone, stearic acid, cacao butter, hydrogenated oils, polysorbate 80, quaternary-ammonium base, sodium lauryl sulfate, glycerin, bentonite, colloidal silicic acid, purified talc, stearate, boric acid powder, polyethylene glycol, colloidal silicic acid, sucrose fatty acids, hardened oil, citric acid, anhydrous citric acid, sodium citrate, sodium citrate dihydrate, anhydrous sodium monohydrogenphosphate, anhydrous sodium dihydrogenphosphate, sodium hydrogen phosphate, iron oxide, beta-carotene, titanium oxide, food colors, copper chlorophyll, riboflavin, ascorbic acid, and various sweeteners.

Those skilled in the art can appropriately select a necessary feature of the composition, such as a dosage form and an optional component.

The form of the composition is not limited to pharmaceutical dosage forms. For example, a $^{13}$C-labeled amino acid may be combined with any foodstuff and formed into solid food, fluid food, or liquid food.

The composition may contain any amount of a $^{13}$C-labeled amino acid without limitation. For example, the amount of a $^{13}$C-labeled amino acid in the composition may be in the range of 1 to 99 wt%. The amount of a $^{13}$C-labeled amino acid administered to a subject may be suitably adjusted for each case, for example, on the basis of factors such as species and condition of the subject, restriction of food intake (fasted/fed), and a form of the composition. When the composition is in oral dosage form or intravenous dosage form, the amount of a single dose (the administered amount) may be adjusted so that the amount of the $^{13}$C-labeled amino acid (active ingredient) is in the range of 0.01 mg/kg body weight to 1 g/kg body weight, preferably 0.02 mg/kg body weight to 0.5 g/kg body weight.

In step (1), the expired air sample may be obtained from the subject at any time point "t" after the administration of a $^{13}$C-labeled amino acid. In other words, the expired air sample may be obtained after the duration from the administration of a $^{13}$C-labeled amino acid to a time point "t". The time point "t" is herein also referred to as expired air collection time "t". Those skilled in the art can determine the expired air collection time "t" appropriately. For example, any time point in the range of about 1 to 120 minutes, about 1 to 60 minutes, about 1 to 40 minutes, about 1 to 20 minutes, about 5 to 60 minutes, about 5 to 40 minutes, about 5 to 20 minutes after the administration of a $^{13}$C-labeled amino acid may be selected. For example, the expired air collection time "t" may be determined by administering a $^{13}$C-labeled amino acid to a mammal of the same species as the subject, collecting expired air samples at plural time points, calculating the ratios of the $^{13}CO_2$ amount to the unlabeled $CO_2$ amount or total $CO_2$ amount for each sample, and selecting the time point at which the ratio is high. For example, the expired air collection time "t" may be in the range of about 1 to 120 minutes or about 5 to 60 minutes after the administration of a $^{13}$C-labeled amino acid.

An expired air sample can be obtained by a conventional method for breath tests known to those skilled in the art. The measurement and analysis of $^{13}CO_2$, unlabeled $CO_2$, and total $CO_2$ contained in an expired air sample is known to those skilled in the art and may be performed by a commonly used analysis method. For example, $^{13}CO_2$ may be measured and analyzed by a commonly used analysis method such as a liquid scintillation counter method, mass spectrometry, infrared spectroscopy, emission spectrometry, or a magnetic resonance spectrum method. Preferably, infrared spectroscopy or mass spectrometry is used.

Step (1) of the method calculates the ratio of the $^{13}CO_2$ amount to the unlabeled $CO_2$ amount or total $CO_2$ amount in an expired air sample. The proportions of each carbon dioxide in an expired air sample may be determined according to a conventional method known to those skilled in the art. An example of such method is described below without limitation.

(1) $\delta^{13}C$ value (‰)

Abundances of isotopes are expressed in terms of isotopic ratio (R) in which the most abundant isotope of the same element is used as the denominator. R value for carbon-13 ($^{13}C$) is expressed by the following formula in which carbon-12 ($^{12}C$) is used as the denominator.

$$R = {}^{13}C / {}^{12}C \qquad \text{(Formula 1)}$$

Since R is a very small numerical value, it is difficult to directly measure it. When a mass spectrometer is used for more accurate quantification, comparison with a standard substance is always performed. The measurement result is represented by $\delta$ value defined by the following formula.

$$\delta^{13}C = ([R_{SAM} / R_{STD}] - 1) \times 1000 \qquad \text{(Formula 2)}$$

$\delta^{13}C$: $\delta^{13}C$ value (‰)
$R_{SAM}$: abundance of $^{13}C$ in sample gas
$R_{STD}$: abundance of $^{13}C$ in standard gas

When carbon dioxide derived from limestone (PDB) is used as standard gas, $R_{STD}$ is $R_{PDB}$ = 0.0112372.

(2) $\Delta^{13}C$ value (‰)

"$\Delta^{13}C$ value (‰)" means a value ($\Delta^{13}C$) obtained by subtracting the $\delta^{13}C$ value before administration of a reagent (i.e., naturally occurring $\delta$ value of $^{13}C$) as a background from the $\delta^{13}C$ value after administration of the reagent, as shown in the following formula.

$$\Delta^{13}C = (\delta^{13}C)t - (\delta^{13}C)0 \qquad \text{(Formula 3)}$$

$\Delta^{13}C$: amount of change in $\delta^{13}C$ value (‰)
$(\delta^{13}C)$ t: $\delta^{13}C$ value at time "t" after reagent administration (‰)
$(\delta^{13}C)$ 0: $\delta^{13}C$ value at time "0" before reagent administration (‰)

(3) $^{13}C$ concentration in expired air (%$^{13}C$: atom%)

The $^{13}C$ concentration in expired air (%$^{13}C$: atom%) is defined by the following formula.

$$\%^{13}C = [{}^{13}C / ({}^{13}C + {}^{12}C)] \times 100$$

To convert the relative value $\delta^{13}C$ defined in (1) into the $^{13}C$ concentration (%) in the total carbon, which is a common concept of concentration, the following method can be used.

First, the numerator and denominator on the right side of the above formula are divided by $^{12}C$, and converted into R based on Formula 1. The following formula is thus obtained.

$$\%^{13}C = [R / (R + 1)] \times 100 \qquad \text{(Formula 4)}$$

The following formula is obtained after $R_{SAM}$ obtained in Formula 2 is substituted into R above and the resulting formula is rearranged. The $^{13}C$ concentration (%$^{13}C$) can be expressed by using the $\delta^{13}C$ value.

$$\%^{13}C = \{ \; [(\delta^{13}C/1000) + 1] \times R_{PDB} \times 100 \} \; / \; \{ \; [[(\delta^{13}C/1000) + 1] \times R_{PDB}] \; + 1 \}$$

(Formula 5)

$\%^{13}C$: $^{13}C$ concentration (atom%)

$\delta^{13}C$: $\delta^{13}C$ value (‰)

$R_{PDB}$: abundance of $^{13}C$ in PDB standard gas = 0.0112372

(4) Amount of change in $^{13}C$ concentration ($\Delta\%^{13}C$)

As defined in the following formula, the amount of change in $^{13}C$ concentration ($\%^{13}C$) in expired air ($\Delta\%^{13}C$) is determined by subtracting the $^{13}C$ concentration at time "0" before administration $[(\%^{13}C)_0]$ from the $^{13}C$ concentration at time "t" after administration $[(\%^{13}C)_t]$.

$$\Delta\%^{13}C = (\%^{13}C)_t - (\%^{13}C)_0$$

(Formula 6)

$\Delta\%^{13}C$: amount of change in $^{13}C$ concentration (atom%)

$(\%^{13}C)_t$: $^{13}C$ concentration at time "t" after reagent administration (atom%)

$(\%^{13}C)_0$: $^{13}C$ concentration at time "0" before reagent administration (atom%)

(5) Relation between $\delta^{13}C$ value (‰) and amount of change in $^{13}C$ concentration ($\Delta\%^{13}C$)

The natural abundance (R) of $^{13}C$ is about 0.011, and even when a labeled reagent is administered, the increased amount in expired air is only about +0.001 to 0.002. Thus, the natural abundance can be regarded as R → 0, and Formula 4, which expresses $\%^{13}C$ by using R, can be approximated by the following formula.

$$\%^{13}C = [R/(R+1)] \times 100 \fallingdotseq R \times 100$$

Using this approximate expression, an approximation that determines the $^{13}C$ concentration, Formula 7, can be obtained as follows: first, $R_{SAM}$ is determined by Formula 2, which defines $\delta^{13}C$, and substituted into R in the above formula, and the resulting formula is rearranged.

$$\%^{13}C = [(\delta^{13}C/1000) + 1] \times R_{PDB} \times 100$$

(Formula 7)

When this is substituted into Formula 6, $\Delta\%^{13}C$ can be calculated from $\Delta^{13}C$, as shown in Formula 8 below.

$$\begin{aligned}\Delta\%^{13}C &= (\%^{13}C)_t - (\%^{13}C)_0 \\ &= \{ [(\delta^{13}C)_t - (\delta^{13}C)_0]/1000 \} \times R_{PDB} \times 100 \\ &= (\Delta^{13}C \times R_{PDB})/10\end{aligned}$$

(Formula 8)

$\Delta\%^{13}C$: amount of change in $^{13}C$ concentration (atom%)

$\Delta^{13}C$: amount of change in $\delta^{13}C$ value (‰)

$R_{PDB}$: abundance of $^{13}C$ in PDB standard gas = 0.0112372

In step (1) of the method, the abundance of carbon dioxide contained in the collected expired air (the ratio of the $^{13}CO_2$ amount to the unlabeled $CO_2$ amount or total $CO_2$ amount) is calculated according to the below-described method as the amount of change in $^{13}C$ concentration ($\Delta\%^{13}C$).

More specifically, the $^{13}C$ concentration in total carbon contained in expired air collected at time "t" after administration of the $^{13}C$-labelled amino acid to a subject ($^{13}C$ concentration in expired air, $^{13}C$ concentration atom %, $(\%^{13}C)_t$) is determined. Similarly, the $^{13}C$ concentration in total carbon contained in expired air collected in advance before administration, preferably at time "0" before administration, ($^{13}C$ concentration in expired air, $^{13}C$ concentration atom %, $(\%^{13}C)_0$) is determined. Further, $(\%^{13}C)_0$ is subtracted from $(\%^{13}C)_t$ according to Formula 6, thereby obtaining the amount of change in the $^{13}C$ concentration ($\Delta\%^{13}C$ (atom%)).

$$^{13}C \text{ concentration (atom\%)} = [^{13}C / (^{13}C + {}^{12}C)] \times 100$$

If necessary, the amount of change in the $^{13}C$ concentration ($\Delta\%^{13}C$) may be converted into $\Delta^{13}C$ value (‰) (amount of change in $\delta^{13}C$ value (‰) or DOB (‰)) by Formula 5 and Formula 3.

The ratio of the $^{13}CO_2$ amount to the unlabeled $CO_2$ amount or total $CO_2$ amount in an expired air sample may be expressed as the area under the curve ($AUC_t$) in a graph showing change in $\Delta\%^{13}C$ or $\Delta^{13}C$(‰) over time. The area under the curve may be calculated according to a commonly used method, which would be easily understood by those skilled in the art. For example, the area under the curve is calculated in a graph showing the change in $\Delta\%^{13}C$ or $\Delta^{13}C$(‰) over time, which has the vertical axis showing the $\Delta\%^{13}C$ or $\Delta^{13}C$ (‰) and the horizontal axis showing the elapsed time after the administration of $^{13}C$-labeled amino acid. For example, $AUC_{t-0}$ may be calculated in the graph, which is the area under the curve from the administration of $^{13}C$-labeled amino acid to the expired air collection time "t". For example, $AUC_{t2-t1}$ may be calculated in the graph, which is the area under the curve from the expired air collection time "$t_1$" to the expired air collection time "$t_2$".

Step (2) of the method compares the ratio calculated in step (1) with a reference value to evaluate the nutriture of the subject. The reference value may be derived from the ratios calculated according to step (1) for a control group, a group of animals of the same species as the subject whose nutriture is known, under the conditions equivalent to those for the subject. For example, the reference value may be derived from the ratios calculated according to step (1) for animals of the same species as the subject which have normal nutriture or undernutrition. The reference value can be determined by calculating the ratios in a control group using the same time "t" as the expired air collection time "t" employed in the calculation of the test value (the ratio of the subject). The reference value may be obtained simultaneously with, in parallel with, before, or after the calculation of the test value. Alternatively, the reference value may be a value predetermined for each species.

The control animal may be an animal of the same species as the subject. Alternatively, the reference value may be determined using the subject itself when its nutriture is known. Examples of animals having normal nutriture include animals that are not fasted and have no disease or its prodrome that is known to cause undernutrition. Examples of animals having undernutrition include fasted animals and animals having a disease known to cause undernutrition. Examples of diseases known to cause undernutrition include hepatic diseases and metabolic diseases, especially liver cirrhosis and diabetes.

The nutriture of a control animal may be evaluated by measuring the respiratory quotient; an animal with a high respiratory quotient may be used as an animal having normal nutriture and an animal with a low respiratory quotient may be used as an animal having undernutrition. According to "Evidence-based Clinical Practice Guidelines for Liver Cirrhosis", energy undernutrition may be diagnosed when the non-protein respiratory quotient is less than 0.85 (Ed. THE JAPANESE SOCIETY OF GASTROENTEROLOGY, Evidence-based Clinical Practice Guidelines for Liver Cirrhosis. Tokyo: Nankodo Co., Ltd., 2010).

In an embodiment, the reference value is derived from the ratios calculated according to step (1) for a control group under the conditions equivalent to those for the subject, wherein the control group is a group of animals of the same species as the subject which have normal nutriture. The subject can be determined to have undernutrition when the ratio calculated for the subject is higher than the reference value and the subject can be determined to have normal nutriture when the ratio is equivalent to or lower than the reference value. In another embodiment, the reference value is derived from the ratios calculated according to step (1) for a control group under the conditions equivalent to those for the subject, wherein the control group is a group of animals of the same species as the subject which have undernutrition. The subject can be determined to have undernutrition when the ratio calculated for the subject is equivalent to or higher than the reference value and the subject can be determined to have better nutriture than that of the control animals when the ratio is lower than the reference value. Alternatively, the reference value may be a ratio calculated according to step (1) for the subject previously. The nutriture of the subject can be determined to have got worse when the ratio calculated for the subject is higher than the reference value and the nutriture of the subject can be determined to have got better when the ratio is lower than the reference value. The degree of the nutriture of the subject also can be determined on the basis of the difference between the test value and the reference value.

The method of the disclosure may be a useful alternative method to conventional methods involving the respiratory quotient measurement. The method enables to determine that a subject has normal nutriture (i.e. the main energy source is carbohydrate) or undernutrition (i.e., the main energy source is lipid and protein). In other words, the method enables to evaluate the energy metabolism status of a subject by determining which nutrient, carbohydrate, lipid, or protein, is used as the main energy source in the subject. The method gives less pain and burdens to the subject and the person conducting the evaluation than the respiratory quotient measurement, and does not need a special and expensive indirect calorimeter. The method thus can precisely determine and evaluate the nutriture of a subject, while decreasing the problems associated with the respiratory quotient measurement.

Furthermore, the conventional respiratory quotient measurement can hardly determine which of lipid and protein is metabolized in the body, since the respiratory quotients for lipid and protein are about 0.7 and about 0.8, respectively. Nevertheless, dietary restriction that allows lipids in the body metabolized and does not allow proteins, which compose organs such as muscles, metabolized is desirable for treating and/or preventing a disease such as obesity or for reducing weight for beauty. The method of the disclosure may be useful for adequate dietary restriction because it enables to determine whether proteins in the body are metabolized.

The method of the disclosure can provide useful information for diagnosing a disease, e.g., a hepatic disease or a metabolic disease, and selecting therapy for such disease. For example, blood albumin levels, respiratory quotients, and BMIs are used as indicators of the energy metabolism in the nutrition assessment of patients with liver cirrhosis. The method may be used in place of the respiratory quotient measurement. For example, the nutriture evaluated by the method and the blood albumin level may be used in combination for determining whether a liver cirrhosis patient has protein energy undernutrition (PEM). In another example, the nutriture evaluated by the method can lead to an appropriate diet therapy in the treatment or prevention of a hepatic or metabolic disease, such as liver cirrhosis, diabetes, borderline diabetes, or insulin resistance. The method may also contribute to the evaluation of the liver glycogen storage or the diagnosis of sarcopenia.

The method of the disclosure may be used for diagnosing sarcopenia or providing information for diagnosing sarcopenia. An aspect of the disclosure thus provides a method of diagnosing sarcopenia of a subject comprising:

(1) calculating the ratio of the $^{13}CO_2$ amount to the unlabeled $CO_2$ amount or total $CO_2$ amount in an expired air sample obtained from the subject to which a $^{13}C$-labeled amino acid was administered; and
(2) comparing the ratio with a reference value to diagnose sarcopenia of the subject.

Sarcopenia is a disease mainly characterized by the decrease in muscle strength and muscle mass. Sarcopenia with no clear cause except for aging is referred to as primary sarcopenia. Secondary sarcopenia includes activity-related sarcopenia, which may be caused, for example, by bedridden state, inactive life or life style, ataxia, or weightlessness; disease-related sarcopenia, which may be associated with a severe organ failure in an organ such as heart, lung, liver, kidney, or brain, an inflammatory disease, a malignant tumor, or a endocrine disease such as diabetes; and nutrition-related sarcopenia, which may be caused by insufficient intake of energy and/or protein associated with malabsorption, a gastrointestinal disease, or anorexia due to drug use. For example, secondary sarcopenia can be caused by diabetes. Sarcopenia may be classified into pre-sarcopenia, sarcopenia, and severe sarcopenia depending on the severity.

The term "diagnosing sarcopenia" includes determining whether the subject has sarcopenia, determining aggravation or improvement of sarcopenia in the subject, determining the effect of treatment of sarcopenia in the subject, or determining severity of sarcopenia in the subject.

Step (1) can be performed according to step (1) of the method of evaluating the nutriture. Step (2) compares the ratio calculated in step (1) with a reference value to diagnose sarcopenia of the subject. The reference value can be set in the same way as the method of evaluating the nutriture. For example, the reference value may be derived from the ratios calculated according to step (1) for a control group, a group of animals of the same species as the subject with sarcopenia or a group of animals of the same species as the subject without sarcopenia, under the feeding condition equivalent to that for the subject.

In an embodiment, the reference value is derived from the ratios calculated according to step (1) for a control group under the conditions equivalent to those for the subject, wherein the control group is a group of animals of the same species as the subject which do not have sarcopenia and are under the feeding condition equivalent to that for the subject. The subject can be determined to have sarcopenia when the ratio calculated for the subject is higher than the reference value and the subject can be determined not to have sarcopenia when the ratio is equivalent to or lower than the reference value. In another embodiment, the reference value is derived from the ratios calculated according to step (1) for a control group under the conditions equivalent to those for the subject, wherein the control group is a group of animals of the same species as the subject which have sarcopenia and are under the feeding condition equivalent to that for the subject. The subject can be determined to have sarcopenia when the ratio calculated for the subject is equivalent to or higher than the reference value and the subject can be determined to have no sarcopenia or less severe sarcopenia when the ratio is lower than the reference value. Alternatively, the reference value may be a ratio calculated according to step (1) for the subject previously under the same conditions. The sarcopenia of the subject can be determined to have aggravated when the ratio calculated for the subject is higher than the reference value and the sarcopenia of the subject can be determined to have improved when the ratio is lower than the reference value. The severity of sarcopenia of the subject also can be determined on the basis of the difference between the test value and the reference value.

An aspect of the disclosure provides a method of evaluating the nutriture of a subject comprising:

(1) administering a $^{13}$C-labeled amino acid to the subject;
(2) obtaining an expired air sample from the subject;
(3) calculating the ratio of the $^{13}CO_2$ amount to the unlabeled $CO_2$ amount or total $CO_2$ amount in the sample; and
(4) comparing the ratio with a reference value to evaluate the nutriture.

An aspect of the disclosure provides a method of diagnosing sarcopenia of a subject comprising:

(1) administering a $^{13}$C-labeled amino acid to the subject;
(2) obtaining an expired air sample from the subject;
(3) calculating the ratio of the $^{13}CO_2$ amount to the unlabeled $CO_2$ amount or total $CO_2$ amount in the sample; and
(4) comparing the ratio with a reference value to diagnose sarcopenia of the subject.

An aspect of the disclosure provides a $^{13}$C-labeled amino acid for use in evaluating the nutriture of a subject.
An aspect of the disclosure provides use of a $^{13}$C-labeled amino acid for manufacturing a composition for evaluating the nutriture of a subject.
An aspect of the disclosure provides a $^{13}$C-labeled amino acid for use in diagnosing sarcopenia of a subject.
An aspect of the disclosure provides use of a $^{13}$C-labeled amino acid for manufacturing a composition for diagnosing sarcopenia of a subject.
For example, the disclosure provides the following embodiments;

[1] A method of evaluating the nutriture of a subject, comprising:

(1) calculating the ratio of the $^{13}CO_2$ amount to the unlabeled $CO_2$ amount or total $CO_2$ amount in an expired air sample obtained from the subject to which a $^{13}$C-labeled amino acid was administered; and
(2) comparing the ratio with a reference value to evaluate the nutriture.

[2] The method according to item 1, wherein the reference value is derived from the ratios calculated according to step (1) for animals of the same species which have normal nutriture.
[3] The method according to item 2, wherein the animals which have normal nutriture are humans having non-protein respiratory quotients of not less than 0.85.
[4] The method according to item 2 or 3, wherein the subject is determined to have undernutrition when the ratio is higher than the reference value and the subject is determined to have normal nutriture when the ratio is equivalent to or lower than the reference value.
[5] The method according to item 1, wherein the reference value is derived from the ratios calculated according to step (1) for animals of the same species which have undernutrition.
[6] The method according to item 5, wherein the animals which have undernutrition are animals having non-protein respiratory quotients of less than 0.85.
[7] The method according to item 5 or 6, wherein the subject is determined to have undernutrition when the ratio is equivalent to or higher than the reference value and the subject is determined to have better nutriture than that of the animals used for the calculation of the reference value when the ratio is lower than the reference value.
[8] The method according to item 1, wherein the reference value is a ratio calculated according to step (1) for the subject previously.
[9] The method according to item 8, wherein the nutriture of the subject is determined to have got worse when the ratio is higher than the reference value and the nutriture of the subject is determined to have got better when the ratio is lower than the reference value.
[10] The method according to any one of items 1 to 9, wherein the subject is in a fasted state.
[11] The method according to any one of items 1 to 9, wherein the subject is in a fed state.
[12] The method according to any one of items 1 to 11, for diagnosing a hepatic disease or a metabolic disease or for providing information for diagnosing a hepatic disease or a metabolic disease.
[13] The method according to any one of items 1 to 12, for diagnosing liver cirrhosis or for providing information for diagnosing liver cirrhosis.
[14] The method according to any one of items 1 to 12, for diagnosing diabetes or for providing information for diagnosing diabetes.

[15] A method of diagnosing sarcopenia of a subject, comprising:

(1) calculating the ratio of the $^{13}CO_2$ amount to the unlabeled $CO_2$ amount or total $CO_2$ amount in an expired air sample obtained from the subject to which a $^{13}C$-labeled amino acid was administered; and
(2) comparing the ratio with a reference value to diagnose sarcopenia of the subject.

[16] The method according to item 15, wherein the reference value is derived from the ratios calculated according to step (1) for animals of the same species which do not have sarcopenia and are under the feeding condition equivalent to that for the subject.

[17] The method according to item 16, wherein the animals which do not have sarcopenia are animals having non-protein respiratory quotients of not less than 0.85.

[18] The method according to item 16 or 17, wherein the subject is diagnosed to have sarcopenia when the ratio is higher than the reference value and the subject is diagnosed not to have sarcopenia when the ratio is equivalent to or lower than the reference value.

[19] The method according to item 15, wherein the reference value is a ratio calculated according to step (1) for the subject previously under the equivalent feeding condition.

[20] The method according to item 19, wherein the sarcopenia of the subject is determined to have aggravated when the ratio is higher than the reference value and the sarcopenia of the subject is determined to have improved when the ratio is lower than the reference value.

[21] The method according to any one of items 15 to 20, wherein the subject is in a fasted state.

[22] The method according to any one of items 15 to 20, wherein the subject is in a fed state.

[23] The method according to any one of items 1 to 22, wherein the $^{13}C$-labeled amino acid is selected from the group consisting of $^{13}C$-labeled alanine, $^{13}C$-labeled glycine, $^{13}C$-labeled serine, $^{13}C$-labeled threonine, $^{13}C$-labeled cysteine, $^{13}C$-labeled tryptophan, $^{13}C$-labeled isoleucine, $^{13}C$-labeled methionine, $^{13}C$-labeled valine, $^{13}C$-labeled aspartic acid, $^{13}C$-labeled asparagine, $^{13}C$-labeled arginine, $^{13}C$-labeled glutamic acid, $^{13}C$-labeled glutamine, $^{13}C$-labeled histidine, $^{13}C$-labeled proline, $^{13}C$-labeled tyrosine, $^{13}C$-labeled phenylalanine, $^{13}C$-labeled leucine, and $^{13}C$-labeled lysine.

[24] The method according to any one of items 1 to 23, wherein the $^{13}C$-labeled amino acid is selected from the group consisting of $^{13}C$-labeled alanine, $^{13}C$-labeled glycine, $^{13}C$-labeled serine, $^{13}C$-labeled threonine, $^{13}C$-labeled cysteine, $^{13}C$-labeled tryptophan, $^{13}C$-labeled isoleucine, $^{13}C$-labeled arginine, $^{13}C$-labeled glutamic acid, $^{13}C$-labeled glutamine, $^{13}C$-labeled histidine, $^{13}C$-labeled proline, $^{13}C$-labeled tyrosine, $^{13}C$-labeled phenylalanine, $^{13}C$-labeled leucine, and $^{13}C$-labeled lysine.

[25] The method according to any one of items 1 to 24, wherein the $^{13}C$-labeled amino acid is selected from the group consisting of $^{13}C$-labeled alanine, $^{13}C$-labeled glutamic acid, and $^{13}C$-labeled leucine.

[26] The method according to any one of items 1 to 25, wherein the $^{13}C$-labeled amino acid is selected from the group consisting of $^{13}C$-labeled alanine and $^{13}C$-labeled glutamic acid.

[27] The method according to any one of items 1 to 24, wherein the $^{13}C$-labeled amino acid is selected from the group consisting of $^{13}C$-labeled alanine, $^{13}C$-labeled glycine, $^{13}C$-labeled serine, $^{13}C$-labeled threonine, $^{13}C$-labeled cysteine, and $^{13}C$-labeled tryptophan.

[28] The method according to any one of items 1 to 27, wherein the $^{13}C$-labeled amino acid is $^{13}C$-labeled alanine.

[29] The method according to any one of items 1 to 24, wherein the $^{13}C$-labeled amino acid is selected from the group consisting of $^{13}C$-labeled glutamic acid, $^{13}C$-labeled glutamine, $^{13}C$-labeled arginine, $^{13}C$-labeled histidine, and $^{13}C$-labeled proline.

[30] The method according to any one of items 1 to 24 and 29, wherein the $^{13}C$-labeled amino acid is $^{13}C$-labeled glutamic acid.

[31] The method according to any one of items 1 to 24, wherein the $^{13}C$-labeled amino acid is selected from the group consisting of $^{13}C$-labeled leucine, $^{13}C$-labeled lysine, $^{13}C$-labeled threonine, $^{13}C$-labeled isoleucine, $^{13}C$-labeled tyrosine, $^{13}C$-labeled phenylalanine, and $^{13}C$-labeled tryptophan.

[32] The method according to any one of items 1 to 25 and 31, wherein the $^{13}C$-labeled amino acid is $^{13}C$-labeled leucine.

[33] The method according to any one of items 1 to 32, wherein the expired air sample is obtained from the subject at a time point "t" after the administration of the $^{13}C$-labeled amino acid.

[34] The method according to item 33, wherein the time point "t" is determined by administering a $^{13}C$-labeled amino acid to a mammal of the same species as the subject, collecting expired air samples at plural time points, calculating the ratios of the $^{13}CO_2$ amount to the unlabeled $CO_2$ amount or total $CO_2$ amount for each sample, and selecting the time point at which the ratio is high.

[35] The method according to item 33 or 34, wherein the time point "t" is in the range of about 1 to 120 minutes after the administration of the $^{13}$C-labeled amino acid.

[36] The method according to any one of items 33 to 35, wherein the time point "t" is in the range of about 1 to 60 minutes after the administration of the $^{13}$C-labeled amino acid.

[37] The method according to any one of items 33 to 36, wherein the time point "t" is in the range of about 1 to 40 minutes after the administration of the $^{13}$C-labeled amino acid.

[38] The method according to any one of items 33 to 37, wherein the time point "t" is in the range of about 1 to 20 minutes after the administration of the $^{13}$C-labeled amino acid.

[39] The method according to any one of items 33 to 36, wherein the time point "t" is in the range of about 5 to 60 minutes after the administration of the $^{13}$C-labeled amino acid.

[40] The method according to any one of items 33 to 37 and 39, wherein the time point "t" is in the range of about 5 to 40 minutes after the administration of the $^{13}$C-labeled amino acid.

[41] The method according to any one of items 33 to 40, wherein the time point "t" is in the range of about 5 to 20 minutes after the administration of the $^{13}$C-labeled amino acid.

[42] The method according to any one of items 1 to 41, wherein the ratio is calculated as $\Delta\%^{13}$C or $\Delta^{13}$C (‰).

[43] The method according to any one of items 1 to 41, wherein the ratio is calculated as AUC.

[44] The method according to any one of items 1 to 43, wherein the subject has a hepatic disease or a metabolic disease.

[45] The method according to any one of items 1 to 44, wherein the subject has liver cirrhosis.

[46] The method according to any one of items 1 to 45, wherein the subject has diabetes.

[47] The method according to any one of items 1 to 46, wherein the subject is under dietary restriction.

[48] The method according to any one of items 1 to 47, further comprising measuring the blood albumin level of the subject and determining whether the subject has protein energy undernutrition (PEM).

[49] A composition comprising a $^{13}$C-labeled amino acid for evaluating the nutriture of a subject.

[50] The composition according to item 49, for diagnosing a hepatic disease or a metabolic disease.

[51] The composition according to item 49, for diagnosing liver cirrhosis.

[52] The composition according to item 49, for diagnosing diabetes.

[53] A composition comprising a $^{13}$C-labeled amino acid diagnosing sarcopenia of a subject.

[54] The composition according to any one of items 49 to 53, wherein the $^{13}$C-labeled amino acid is selected from the group consisting of $^{13}$C-labeled alanine, $^{13}$C-labeled glycine, $^{13}$C-labeled serine, $^{13}$C-labeled threonine, $^{13}$C-labeled cysteine, $^{13}$C-labeled tryptophan, $^{13}$C-labeled isoleucine, $^{13}$C-labeled methionine, $^{13}$C-labeled valine, $^{13}$C-labeled aspartic acid, $^{13}$C-labeled asparagine, $^{13}$C-labeled arginine, $^{13}$C-labeled glutamic acid, $^{13}$C-labeled glutamine, $^{13}$C-labeled histidine, $^{13}$C-labeled proline, $^{13}$C-labeled tyrosine, $^{13}$C-labeled phenylalanine, $^{13}$C-labeled leucine, and $^{13}$C-labeled lysine.

[55] The composition according to any one of items 49 to 54, wherein the $^{13}$C-labeled amino acid is selected from the group consisting of $^{13}$C-labeled alanine, $^{13}$C-labeled glycine, $^{13}$C-labeled serine, $^{13}$C-labeled threonine, $^{13}$C-labeled cysteine, $^{13}$C-labeled tryptophan, $^{13}$C-labeled isoleucine, $^{13}$C-labeled arginine, $^{13}$C-labeled glutamic acid, $^{13}$C-labeled glutamine, $^{13}$C-labeled histidine, $^{13}$C-labeled proline, $^{13}$C-labeled tyrosine, $^{13}$C-labeled phenylalanine, $^{13}$C-labeled leucine, and $^{13}$C-labeled lysine.

[56] The composition according to any one of items 49 to 55, wherein the $^{13}$C-labeled amino acid is selected from the group consisting of $^{13}$C-labeled alanine, $^{13}$C-labeled glutamic acid, and $^{13}$C-labeled leucine.

[57] The composition according to any one of items 49 to 56, wherein the $^{13}$C-labeled amino acid is selected from the group consisting of $^{13}$C-labeled alanine and $^{13}$C-labeled glutamic acid.

[58] The composition according to any one of items 49 to 55, wherein the $^{13}$C-labeled amino acid is selected from the group consisting of $^{13}$C-labeled alanine, $^{13}$C-labeled glycine, $^{13}$C-labeled serine, $^{13}$C-labeled threonine, $^{13}$C-labeled cysteine, and $^{13}$C-labeled tryptophan.

[59] The composition according to any one of items 49 to 58, wherein the $^{13}$C-labeled amino acid is $^{13}$C-labeled alanine.

[60] The composition according to any one of items 49 to 55, wherein the $^{13}$C-labeled amino acid is selected from the group consisting of $^{13}$C-labeled glutamic acid, $^{13}$C-labeled glutamine, $^{13}$C-labeled arginine, $^{13}$C-labeled histidine, and $^{13}$C-labeled proline.

[61] The composition according to any one of items 49 to 55 and 60, wherein the $^{13}$C-labeled amino acid is $^{13}$C-labeled glutamic acid.

[62] The composition according to any one of items 49 to 55, wherein the $^{13}$C-labeled amino acid is selected from the group consisting of $^{13}$C-labeled leucine, $^{13}$C-labeled lysine, $^{13}$C-labeled threonine, $^{13}$C-labeled isoleucine, $^{13}$C-labeled tyrosine, $^{13}$C-labeled phenylalanine, and $^{13}$C-labeled tryptophan.

[63] The composition according to any one of items 49 to 56 and 62, wherein the $^{13}$C-labeled amino acid is $^{13}$C-labeled leucine.

[64] The composition according to any one of items 49 to 63, wherein the composition is in oral dosage form.

[65] The composition according to any one of items 49 to 63, wherein the composition is in parenteral dosage form.

The entire contents of the documents cited herein are incorporated herein by reference.

The embodiments described above are non-limiting and may be modified without deviating from the scope of the invention as defined by the appended claims. The following example does not restrict or limit the invention and is for illustrative purposes only.

EXAMPLE 1

Animals

Zucker Diabetic Fatty Rat (ZDF-Lepr$^{fa}$/CrlCrlj) (CHARLES RIVER LABORATORIES JAPAN, INC., Kanagawa, Japan) was used.

ZDF fatty rat, which has a homozygous mutation causing obesity in leptin receptor gene (Lepr$^{fa}$/Lepr$^{fa}$), was used as a model animal for diabetes.

ZDF Lean rat has a homozygous dominant leptin receptor gene (+/+) or a heterozygote (Lepr$^{fa}$/+).

Comparison of results between respiratory quotient measurement and breath test using $^{13}$C-amino acids in ZDF lean rats

(1) Respiratory quotient measurement

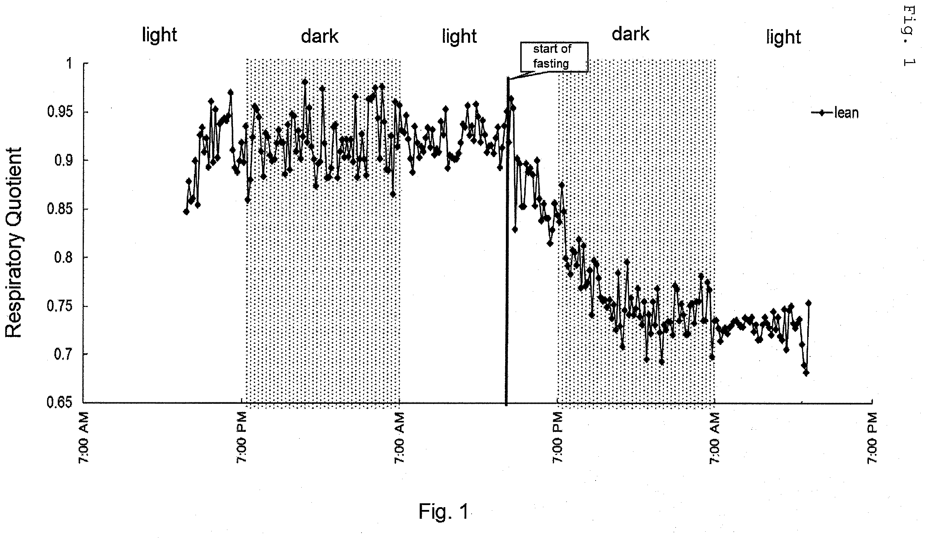

Male ZDF lean rats (35 to 36 weeks old, n = 2) were used. The rats were maintained on water ad libitum throughout the test period. As shown in Fig. 1, the rats were in a fed state from 19:00 on Day 1 to 16:00 on Day 2 of the test and in a fasted state from 16:00 on Day 2 to 13:00 on Day 3. In the fed state, animal feed (trade name CRF-1, a product made by Oriental Yeast Co., Ltd.) was provided ad libitum. The dark periods were from 19:00 to 7:00 and the light periods were from 7:00 to 19:00. The respiratory quotients of the rats were measured at each time point with a mass spectrometer for analyzing biogas ("Oxymax" Columbus Instruments International). The results are shown in Fig. 1. In the fed state, the respiratory quotients were in the range of about 0.9 to 0.95 through the night and day, though activity of rats is high in night and low in day. In the fasted state, the respiratory quotients were in the range of about 0.7 to 0.75. This suggests that fasting decreases the glycogen storage in the liver and lipids and/or proteins are burnt as the energy source.

(2) Breath test using $^{13}$C-amino acid

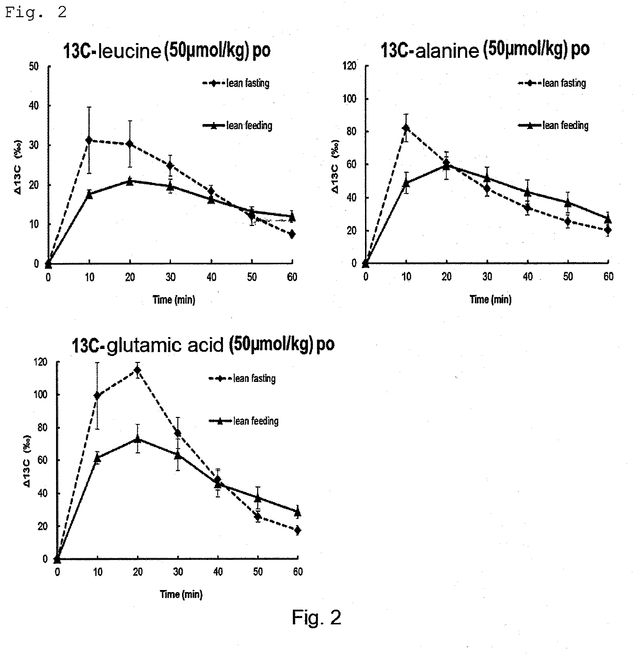

ZDF lean rats were placed in the fed/fasted state and the light-dark cycle as described above. 1-$^{13}$C-leucine, 1-$^{13}$C-alanine, or 1-$^{13}$C-glutamic acid (Cambridge Isotope Laboratories, Inc (Andover, MA, USA)) dissolved in physiological saline was administered orally at 50 $\mu$mol/kg each (n = 4 for each amino acid) at 13:00 of Day 2 in the fed state or 13:00 of Day 3 in the fasted state. Expired air samples were collected before the administration (0 minutes) and at several time points up to 60 minutes after the administration and $\Delta^{13}$C (‰) values were measured using a mass spectrometer for breath analysis ("ABCA" Sercon). The results are shown in Fig. 2. The $\Delta^{13}$C (‰) values at 10 minutes after the administration were higher in the fasted state than in the fed state for all the amino acids, and this tendency continued for about 45, 20, and 40 minutes for 1-$^{13}$C-leucine, 1-$^{13}$C-alanine, and 1-$^{13}$C-glutamic acid, respectively. This indicates that the amino acid metabolism is increased in the fasted state, suggesting that fasting decreases the glycogen storage in the liver and proteins are burnt as the energy source.

(3) Conclusion

The results reflected the nutriture, i.e, the fasted state or the fed state, in both of the respiratory quotient measurement and the breath test using a $^{13}$C-amino acid. This suggests that the breath test using a $^{13}$C-amino acid may be used as an alternative to the respiratory quotient measurement.

Example 2

Respiratory quotient measurement, breath test using $^{13}$C-alanine, and measurement of albumin level in ZDF fatty rats

(1) Respiratory quotient measurement

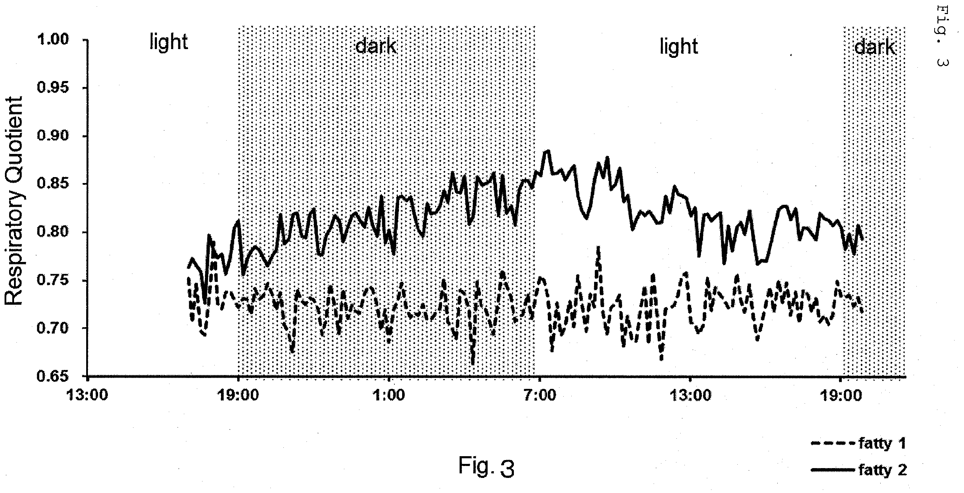

Two 36-week-old male ZDF fatty rats (diabetes model), F1 and F2, were used. F1 was expected to have developed diabetic sarcopenia, which decreases muscle strength and muscle mass, because it had a low body weight, frailty, and low activity. The rats were maintained on water and animal feed (trade name CRF-1, a product made by Oriental Yeast Co., Ltd.) ad libitum throughout the test period. The dark periods were from 19:00 to 7:00 and the light periods were from 7:00 to 19:00. The respiratory quotients of the rats were measured at each time point with a mass spectrometer for analyzing biogas ("Oxymax" Columbus Instruments International). The results are shown in Fig. 3. The respiratory quotients of F1 rat were in the range of about 0.7 to 0.75 and the respiratory quotients of F2 rat were in the range of about 0.75 to 0.85. The respiratory quotients were lower in F1 rat than in F2 rat, though they were fed. The results agree with that F1 rat, which is suspected to have sarcopenia, is considered to use proteins from muscles as the energy source.

(2) Breath test using $^{13}$C-alanine

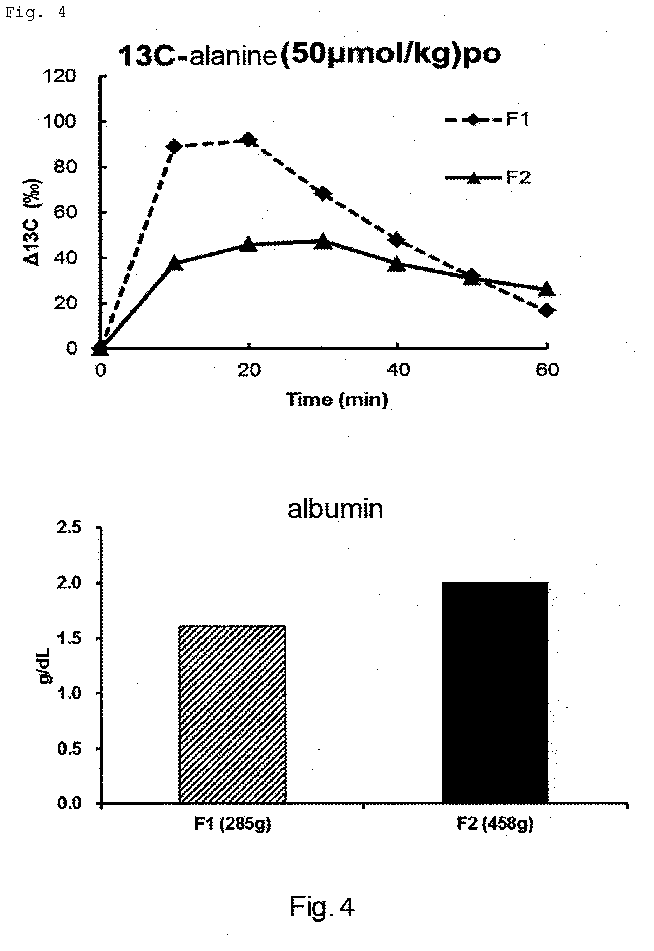

ZDF fatty rats F1 and F2 mentioned above were placed in the light-dark cycle as described above. 1-$^{13}$C-alanine dissolved in physiological saline was administered orally at 50 $\mu$mol/kg. Expired air samples were collected before the administration (0 minutes) and at several time points up to 60 minutes after the administration and $\Delta^{13}$C (‰) values were measured using a mass spectrometer for breath analysis ("ABCA" Sercon). The results are shown in Fig. 4. The $\Delta^{13}$C (‰) values were higher in F1 rat than in F2 rat from the start of the administration to about 50 minutes after the administration. This indicates that the amino acid metabolism is increased in F1 rat. The results agree with that F1 rat, which is suspected to have sarcopenia, is considered to use proteins from muscles as the energy source.

(3) Conclusion

The results reflected the nutriture, i.e, the glycogen storage caused by the disease, in both of the respiratory quotient measurement and the breath test using a $^{13}$C-amino acid. This suggests that the breath test using a $^{13}$C-amino acid may be used as an alternative to the respiratory quotient measurement.

Example 3

Respiratory quotient measurement, breath test using $^{13}$C-glutamic acid, and measurement of albumin level in ZDF fatty rats

(1) Respiratory quotient measurement

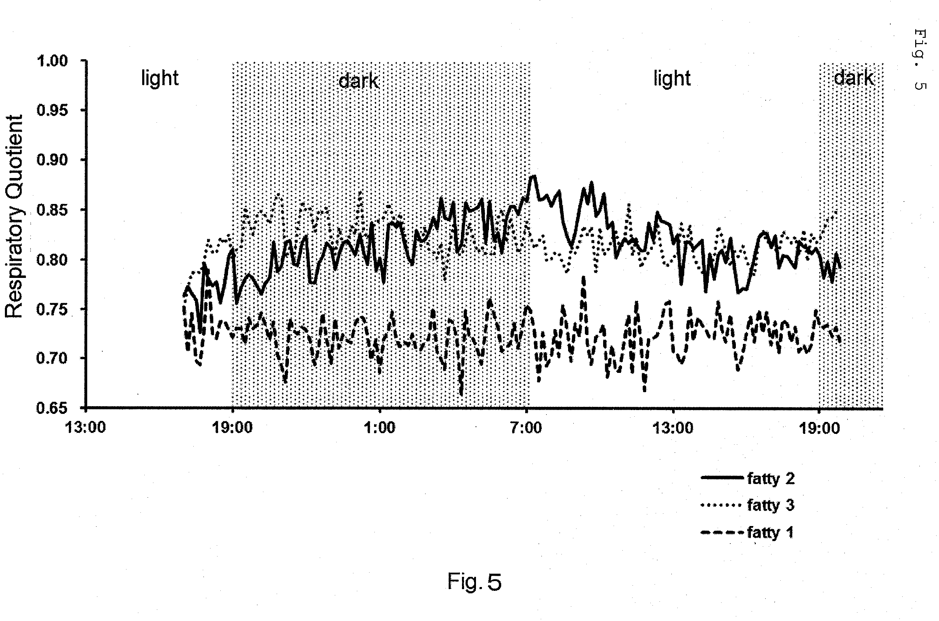

Three 36-week-old male ZDF fatty rats (diabetes model), F1, F2, and F3, were used. F1 was expected to have developed diabetic sarcopenia, which decreases muscle strength and muscle mass, because it had a low body weight, frailty, and low activity. The rats were maintained on water and animal feed (trade name CRF-1, a product made by Oriental Yeast Co., Ltd.) ad libitum throughout the test period. The dark periods were from 19:00 to 7:00 and the light periods were from 7:00 to 19:00. The respiratory quotients of the rats were measured at each time point with a mass spectrometer for analyzing biogas ("Oxymax" Columbus Instruments International). The results are shown in Fig. 5. The respiratory quotients of F1 rat were in the range of about 0.7 to 0.75 and the respiratory quotients of F2 and F3 rats were in the range of about 0.75 to 0.85. The respiratory quotients were lower in F1 rat than in F2 and F3 rats, though they were fed. The results agree with that F1 rat, which is suspected to have sarcopenia, is considered to use proteins from muscles as the energy source.

(2) Breath test using $^{13}$C-glutamic acid

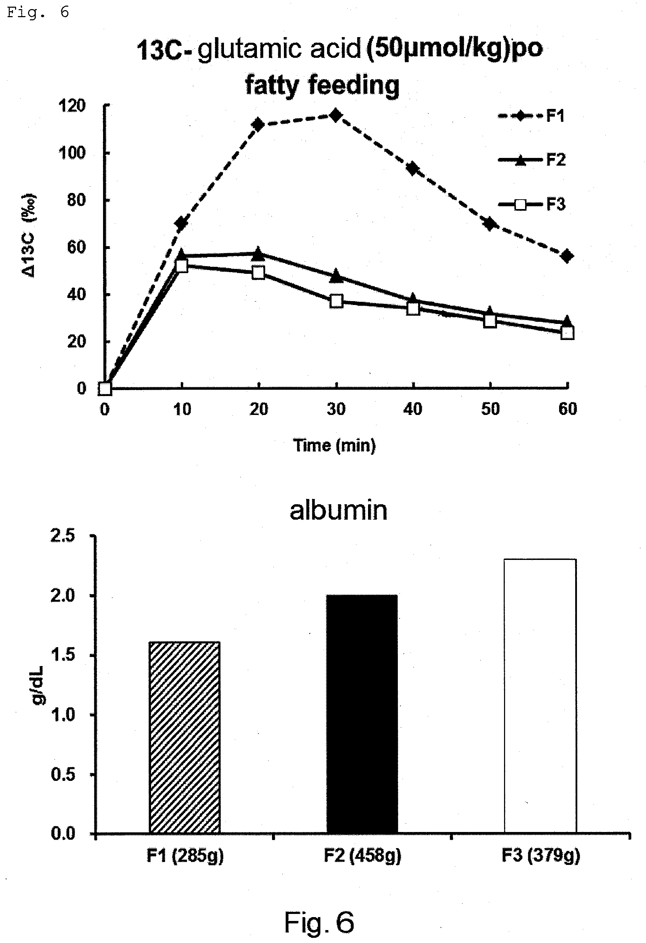

ZDF fatty rats F1, F2, and F3 mentioned above were placed in the light-dark cycle as described above. 1-$^{13}$C-glutamic acid dissolved in physiological saline was administered orally at 50 $\mu$mol/kg. Expired air samples were collected before the administration (0 minutes) and at several time points up to 60 minutes after the administration and $\Delta^{13}$C (‰) values were measured using a mass spectrometer for breath analysis ("ABCA" Sercon) . The results are shown in Fig. 6. The $\Delta^{13}$C (‰) values were higher in F1 rat than in F2 and F3 rats from the start of the administration to the end of the measurement. This indicates that the amino acid metabolism is increased in F1 rat. The results agree with that F1 rat, which is suspected to have sarcopenia, is considered to use proteins from muscles as the energy source.

(3) Conclusion

The results reflected the nutriture, i.e, the glycogen storage caused by the disease, in both of the respiratory quotient measurement and the breath test using a $^{13}$C-amino acid. This suggests that the breath test using a $^{13}$C-amino

acid may be used as an alternative to the respiratory quotient measurement.

INDUSTRIAL APPLICABILITY

The disclosure enables to evaluate the real-time nutriture of a subject simply and rapidly, providing an alternative method to the respiratory quotient measurement. The method may be used for the same purpose as the conventional respiratory quotient measurement.

**Claims**

1.  A method of evaluating the nutriture of a subject, comprising:

    (1) calculating the ratio of the $^{13}CO_2$ amount to the unlabeled $CO_2$ amount or total $CO_2$ amount in an expired air sample obtained from the subject to which a $^{13}C$-labeled amino acid was administered; and
    (2) comparing the ratio with a reference value to evaluate the nutriture.

2.  The method according to claim 1, wherein the reference value is derived from the ratios calculated according to step (1) for animals of the same species which have normal nutriture.

3.  The method according to claim 2, wherein the subject is determined to have undernutrition when the ratio is higher than the reference value and the subject is determined to have normal nutriture when the ratio is equivalent to or lower than the reference value.

4.  The method according to any one of claims 1 to 3, wherein the $^{13}C$-labeled amino acid is selected from the group consisting of $^{13}C$-labeled alanine, $^{13}C$-labeled glycine, $^{13}C$-labeled serine, $^{13}C$-labeled threonine, $^{13}C$-labeled cysteine, and $^{13}C$-labeled tryptophan.

5.  The method according to any one of claims 1 to 3, wherein the $^{13}C$-labeled amino acid is selected from the group consisting of $^{13}C$-labeled glutamic acid, $^{13}C$-labeled glutamine, $^{13}C$-labeled arginine, $^{13}C$-labeled histidine, and $^{13}C$-labeled proline.

6.  The method according to any one of claims 1 to 3, wherein the $^{13}C$-labeled amino acid is selected from the group consisting of $^{13}C$-labeled leucine, $^{13}C$-labeled lysine, $^{13}C$-labeled threonine, $^{13}C$-labeled isoleucine, $^{13}C$-labeled tyrosine, $^{13}C$-labeled phenylalanine, and $^{13}C$-labeled tryptophan.

7.  The method according to any one of claims 1 to 3, wherein the $^{13}C$-labeled amino acid is selected from the group consisting of $^{13}C$-labeled alanine, $^{13}C$-labeled glutamic acid, and $^{13}C$-labeled leucine.

8.  The method according to any one of claims 1 to 7, wherein the subject has a hepatic disease or a metabolic disease.

9.  The method according to any one of claims 1 to 8, wherein the subject has liver cirrhosis.

10. The method according to any one of claims 1 to 9, wherein the subject has diabetes.

11. A composition comprising a $^{13}C$-labeled amino acid for evaluating the nutriture of a subject.

12. The composition according to claim 11, wherein the $^{13}C$-labeled amino acid is selected from the group consisting of $^{13}C$-labeled alanine, $^{13}C$-labeled glycine, $^{13}C$-labeled serine, $^{13}C$-labeled threonine, $^{13}C$-labeled cysteine, and $^{13}C$-labeled tryptophan.

13. The composition according to claim 11, wherein the $^{13}C$-labeled amino acid is selected from the group consisting of $^{13}C$-labeled glutamic acid, $^{13}C$-labeled glutamine, $^{13}C$-labeled arginine, $^{13}C$-labeled histidine, and $^{13}C$-labeled proline.

14. The composition according to claim 11, wherein the $^{13}C$-labeled amino acid is selected from the group consisting of $^{13}C$-labeled leucine, $^{13}C$-labeled lysine, $^{13}C$-labeled threonine, $^{13}C$-labeled isoleucine, $^{13}C$-labeled tyrosine, $^{13}C$-labeled phenylalanine, and $^{13}C$-labeled tryptophan.

**15.** The composition according to claim 11, wherein the $^{13}C$-labeled amino acid is selected from the group consisting of $^{13}C$-labeled alanine, $^{13}C$-labeled glutamic acid, and $^{13}C$-labeled leucine.

**16.** A method of diagnosing sarcopenia of a subject, comprising:

(1) calculating the ratio of the $^{13}CO_2$ amount to the unlabeled $CO_2$ amount or total $CO_2$ amount in an expired air sample obtained from the subject to which a $^{13}C$-labeled amino acid was administered; and
(2) comparing the ratio with a reference value to diagnose sarcopenia of the subject.

**17.** The method according to claim 16, wherein the reference value is derived from the ratios calculated according to step (1) for animals of the same species which do not have sarcopenia and are under the feeding condition equivalent to that for the subject.

**18.** The method according to claim 17, wherein the subject is diagnosed to have sarcopenia when the ratio is higher than the reference value and the subject is diagnosed not to have sarcopenia when the ratio is equivalent to or lower than the reference value.

Fig. 1

Fig. 1

Fig. 2

Fig. 2

Fig. 3

Fig. 3

Fig. 4

**13C-alanine(50µmol/kg)po**

**albumin**

Fig. 4

EP 3 546 936 A1

Fig. 5

Fig. 5

Fig. 6

Fig. 6

## INTERNATIONAL SEARCH REPORT

| International application No. |
|---|
| PCT/JP2017/042150 |

**A. CLASSIFICATION OF SUBJECT MATTER**
Int. Cl.  G01N33/497(2006.01)i, G01N33/66(2006.01)i

According to International Patent Classification (IPC) or to both national classification and IPC

**B. FIELDS SEARCHED**

Minimum documentation searched (classification system followed by classification symbols)
Int. Cl.  G01N33/497, G01N33/66

Documentation searched other than minimum documentation to the extent that such documents are included in the fields searched
Published examined utility model applications of Japan    1922-1996
Published unexamined utility model applications of Japan  1971-2018
Registered utility model specifications of Japan          1996-2018
Published registered utility model applications of Japan  1994-2018

Electronic data base consulted during the international search (name of data base and, where practicable, search terms used)
JSTPlus/JMEDPlus/JST7580 (JDreamIII)

**C. DOCUMENTS CONSIDERED TO BE RELEVANT**

| Category* | Citation of document, with indication, where appropriate, of the relevant passages | Relevant to claim No. |
|---|---|---|
| Y | WO 2016/006601 A1 (OTSUKA PHARMACEUTICAL CO., LTD.) 14 January 2016, entire text, all drawings, in particular, claims & US 2017/0199172 A1, entire text, all drawings, in particular, see claims, etc. | 1-10, 16-18 |
| Y/X | JP 11-209309 A (TOKYO GAS CO., LTD.) 03 August 1999, entire text, all drawings, in particular, claims, paragraphs [0010]-[0023], [0027]-[0056] & US 6509002 B1, entire text, all drawings, in particular, see claims, column 6, line 3 to column 7, line 44, column 10, line 29 to column 11, line 67, column 13, line 5 to column 20, line 10 & EP 913161 A2 | 1-10, 16-18/ 11-15 |

☒  Further documents are listed in the continuation of Box C.    ☐  See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| | |

| Name and mailing address of the ISA/ | Authorized officer |
|---|---|
| Japan Patent Office | |
| 3-4-3, Kasumigaseki, Chiyoda-ku, | |
| Tokyo 100-8915, Japan | Telephone No. |

Form PCT/ISA/210 (second sheet) (January 2015)

**INTERNATIONAL SEARCH REPORT**

International application No.

PCT/JP2017/042150

C (Continuation).    DOCUMENTS CONSIDERED TO BE RELEVANT

| Category* | Citation of document, with indication, where appropriate, of the relevant passages | Relevant to claim No. |
|---|---|---|
| Y | 荒木 厚, 糖尿病におけるサルコペニアの意義,医学のあゆみ, 01 March 2014, vol. 248, no. 9, pp. 733-737 non-official translation (ARAKI, Atsushi, Significance of sarcopenia in diabetes, Journal of Clinical and Experimental Medicine (IGAKU NO AYUMI)) | 16-18 |

Form PCT/ISA/210 (continuation of second sheet) (January 2015)

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2016229121 A
- WO 2016006601 A
- WO 2014030650 A
- WO 2014142248 A

**Non-patent literature cited in the description**

- **YASUHITO SASAKI.** 5.1 Antei Doitai no Rinsho Shindan heno Oyo [5.1 Application of Stable Isotopes in Clinical Diagnosis. *Kagaku no Ryoiki [Journal of Japanese Chemistry,* 1975, vol. 107, 149-163
- **NANKODO ; KAJIWARA.** *RADIOISOTOPES,* 1992, vol. 41, 45-48
- Evidence-based Clinical Practice Guidelines for Liver Cirrhosis. Nankodo Co., Ltd, 2010